# EUROPEAN PATENT APPLICATION

(11) **EP 4 600 370 A1**
(43) Date of publication of application: **13.08.2025**
(21) Application number: 24157075.3
(22) Date of filing: 12.02.2024
(51) Int. Cl.: C12P 19/34, C12N 9/14, C12P 19/36

(54) **NUDE.1 AS A DECAPPING ENZYME FOR COFACTOR- AND "CANONICALLY"-CAPPED RNA SPECIES**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a method for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein the method comprises contacting the 5'-dinucleotide-capped nucleic acid molecule with (I) an enzyme being capable of cleaving the diphosphate, triphosphate or tetraphosphate linkage, wherein the enzyme (a) comprises or consists of the amino acid sequence of SEQ ID NO: 21, (b) comprises or consists of an amino acid sequence being at least 80%, preferably at least 90% and most preferably at least 95% identical to SEQ ID NO: 21, (c) comprises or consists of the amino acid sequence being encoded by the nucleotide sequence of SEQ ID NO: 17, or (d) comprises or consists of an amino acid sequence being encoded by a nucleotide sequence being at least 80%, preferably at least 90% and most preferably at least 95% identical to SEQ ID NO: 17; (II) a nucleic acid molecule, preferably a vector, encoding in expressible form the enzyme of (I); and/or (III) a host cell comprising the nucleic acid molecule, preferably the vector of (II).

## Description

The present invention relates to a method for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein the method comprises contacting the 5'-dinucleotide-capped nucleic acid molecule with (I) an enzyme being capable of cleaving the diphosphate, triphosphate or tetraphosphate linkage, wherein the enzyme (a) comprises or consists of the amino acid sequence of SEQ ID NO: 21, (b) comprises or consists of an amino acid sequence being at least 80%, preferably at least 90% and most preferably at least 95% identical to SEQ ID NO: 21, (c) comprises or consists of the amino acid sequence being encoded by the nucleotide sequence of SEQ ID NO: 17, or (d) comprises or consists of an amino acid sequence being encoded by a nucleotide sequence being at least 80%, preferably at least 90% and most preferably at least 95% identical to SEQ ID NO: 17; (II) a nucleic acid molecule, preferably a vector, encoding in expressible form the enzyme of (I); and/or (III) a host cell comprising the nucleic acid molecule, preferably the vector of (II).

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Besides its four canonical nucleotides, RNA molecules can be decorated with diverse chemical modifications at its termini or within the RNA body ^{1,2}. For a long time, 5'-cap-like structures on RNA were thought to exist only in eukaryotes in form of the N7-methylguanosine cap (m7G-cap). However, it is known today that redox cofactors including flavin adenine dinucleotide (FAD), dephospho-coenzyme A (dpCoA) or nicotinamide adenine dinucleotide (NAD) equip the 5'-end of RNAs as cap-like structures ³⁻⁶. NAD-capped RNAs (NAD-RNAs) are the best studied types of these cofactor-capped RNA species regarding their identity and biosynthesis ⁷. Also, dinucleotides composed of canonical nucleosides (N) connected by a series of phosphates (pn) that are termed NpnN can cap RNA molecules (Potužník et al., Angew Chem Int Ed Engl. (2023); e202314951 and Hudeček et al., Nat Commun 11, 1052 (2020)). For instance, Ap4A serves as a cap-like structure on bacterial and eukaryotic RNA.

First evidence for the existence of NAD-caps *in vivo* was obtained via LC-MS analysis of total RNA ⁵, the identity of NAD-RNAs, however, remained unknown due to the required degradation of total RNA prior to analysis. Therefore, NAD captureSeq was developed which allows for the chemoenzymatic capture, enrichment, and sequencing of NAD-RNAs from total RNA ⁴. Briefly, NAD-RNA is specifically enzymatically labelled with an alkyne-group, which is subsequently fused to an azide-modified biotin group via copper-catalyzed "click" chemistry. Then, the biotinylated NAD-RNAs can be enriched from total RNA and sequenced. To cope with RNA degradation due to copper-catalyzed "clicking" of biotin, strain-promoted (copper-free) "click" chemistry ⁸ and Nanopore sequencing have been used to optimize NAD captureSeq ⁹. This enhances RNA integrity and enables the sequencing of full-length NAD-RNAs ⁹. Overall, based on the NAD captureSeq technology, NAD-RNAs have been identified in bacteria ^{10,11}, including *Escherichia coli ^{4,9}*, archaea ^{11,13} and eukaryotes ^{8,14} highlighting the universal existence of this cofactor-capped RNA species ⁷.

The predominant - and to this day the only known - biosynthesis mechanism of NAD-RNAs involves the RNA Polymerase (RNAP) which uses NAD as a non-canonical initiating nucleotide (NCIN). Instead of ATP, it incorporates NAD (with an adenosine moiety involved in base pairing) at the 5'-end of RNA *ab initio* during transcription initiation ^{7,15}. In bacteria, the NAD-cap provides a stabilizing effect to the RNA, whilst it was found to promote RNA decay in eukaryotes. NAD-RNA decapping and subsequent degradation can occur via two pathways. NAD-cap hydrolysis by Nudix hydrolases cleaves the pyrophosphate group within the NAD-cap releasing 5'-monophosphorylated RNA which can be subjected to cleavage afterwards. DeNADding refers to the removal of the entire NAD-cap and has only been described in eukaryotes to this day. Apart from that the functions of NAD-RNAs are poorly understood.

In order to study potential roles of NAD-RNAs it may seem promising to look beyond typical model organisms. Pathogen-host interactions extent beyond the classical world of pro- and eukaryotes. However, NAD-RNAs have only been indicated to exist in infection by eukaryotic virus - the Dengue virus - using LC-MS ⁶. The identity of the NAD-RNAs in this context remains elusive though.

NAD-RNAs have been initially discovered in *E. coli* ^{4,16} which is - among others - infected by bacteriophage T4 (T4 phage) ¹⁷. It infects *E. coli* by injecting its 169 kb dsDNA genome and protein effectors in the host cell ¹⁷. Then, a fine-tuned infection program begins to take over the host's gene expression machinery including the RNAP and ribosomes to express its genes ¹⁷. Thereby, the T4 phage enables to create the ideal environment in order to express its genes starting with host reprogramming factors in early, replication genes in middle and structural phage proteins in late phase of infection ^{18,19}. Moreover, the phage triggers the shut-off of host gene transcription and RNA degradation ¹⁷⁻¹⁹. Albeit these processes have been basically understood, detailed mechanistic understanding, e.g. how the RNA degradation machinery distinguishes between phage and host RNA remains elusive. Epitranscriptomic mechanisms may provide a regulatory layer to achieve these characteristics. However, epitranscriptomics have not yet been applied to phage research at all. Just recently, NAD-RNAs have been found to serve as substrates for an ADP-ribosyltransferase (ART) from bacteriophage T4 (T4 phage) ²⁰. The ART ModB uses NAD-RNAs in an RNAylation reaction, by which it covalently attaches NAD-RNAs to ribosomal proteins of its host *Escherichia coli* including ribosomal proteins S1 and L2 ²⁰. This may provide a mean for the T4 phage to regulate gene expression during T4 phage infection on the translational level. Further, it indicates a distinct biological function of NAD-RNAs, namely activation of the RNA for the enzymatic transfer to a target protein ²⁰. Apart from this role of NAD-RNAs in RNAylation of host proteins during infection, the abundance, identity, biosynthesis and degradation of NAD-RNAs during T4 phage infection are poorly understood.

In the present application, the time-resolved dual NAD-capped epitranscriptome of T4 phage infection is studied for the first time - namely the parallel and comprehensive NAD-capped transcripts of the T4 phage and its host *E. coli* during infection. In this connection the role of the T4 phage Nudix hydrolase NudE.1 in the hydrolysis of NAD, NAD-RNAs and related protein modifications has been revealed. The data shows that NudE.1 exhibits promiscuous activity on these cofactor-derived biomolecules during infection, thereby providing an important regulatory function during T4 phage infection. Further, NudE.1 can be a useful enzyme for applications in synthetic biology. Hence, the present application comprises the first study of epitranscriptomic modifications in phages and identification of virus-derived NAD-capped transcripts. Most importantly, it is shown herein that the T4 phage Nudix hydrolase NudE.1 as a phage enzyme is directly involved in decapping of NAD-RNAs *in vivo.* Thereby, the application provides novel means and methods for decapping a 5'-dinucleotide-capped nucleic acid molecule.

Accordingly, the present invention relates in first aspect to a method for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein the method comprises contacting the dinucleotide-capped nucleic acid molecule with (I) an enzyme being capable of cleaving the diphosphate, triphosphate or tetraphosphate linkage, wherein the enzyme (a) comprises or consists of the amino acid sequence of SEQ ID NO: 21, (b) comprises or consists of an amino acid sequence being at least 80%, preferably at least 90% and most preferably at least 95% identical to SEQ ID NO: 21, (c) comprises or consists of the amino acid sequence being encoded by the nucleotide sequence of SEQ ID NO: 17, or (d) comprises or consists of an amino acid sequence being encoded by a nucleotide sequence being at least 80%, preferably at least 90% and most preferably at least 95% identical to SEQ ID NO: 17; (II) a nucleic acid molecule, preferably a vector, encoding in expressible form the enzyme of (I); and/or (III) a host cell comprising the nucleic acid molecule, preferably the vector of (II).

A 5'-dinucleotide-capped nucleic acid molecule, wherein the dinucleotide is linked to the nucleic acid molecule via a phosphodiester designates a nucleic acid sequence, wherein a dinucleotide is linked to the 5' end of the nucleic acid sequence via a phosphodiester linkage.

A dinucleotide is an organic compound comprised of two nucleotides (as opposed to other types, such as mononucleotides and trinucleotides). A nucleotide is an organic compound made up of three subunits: a nucleobase (either a purine or a pyrimidine), a five-carbon sugar (pentose), and a phosphate group. The sugar component may either be ribose or deoxyribose. The ribose sugar is the sugar component of the nucleotides that make up RNA. The deoxyribose sugar is the sugar component of DNA. Each phosphate group connects the sugar rings of two adjacent nucleotide monomers. The phosphate groups and the sugar moieties form the backbone of a nucleic acid. The directionality of the chain runs from 5'-end to 3'-end. Hence, a nucleobase is a nitrogen-containing biological compound that forms nucleosides. Nucleosides are components of nucleotides, with all of these monomers constituting the basic building blocks of nucleic acids. Examples of dinucleotide will be provided herein below.

A dinucleotide comprising a diphosphate, triphosphate or tetraphosphate linkage between the two nucleotides of the dinucleotide comprise two, three or four phosphate groups between the two nucleotides. An example of dinucleotide comprising a diphosphate linkage between the two nucleotides is nicotinamidadenindinucleotide (NAD). An example a dinucleotide comprising a triphosphate linkage between the two nucleotides is 7-methyl-guanosine-5'-triphosphate-5'-guanosine (also known as 7-methyl-gpppG). An example a dinucleotide comprising a tetraphosphate linkage between the two nucleotides is diadenosine tetraphosphate (also known as Ap4A).

The term "nucleic acid sequence" (also called "nucleic acid molecule" herein) in accordance with the present invention includes DNA, such as double or single stranded DNA and RNA. The nucleic acid sequence is preferably single stranded, such as single stranded DNA and RNA and is most preferably single-stranded RNA. In this regard, "DNA" (deoxyribonucleic acid) means any chain or sequence of the chemical building blocks adenine (A), guanine (G), cytosine (C) and thymine (T), called nucleotide bases that are linked together on a deoxyribose sugar backbone. DNA can have one strand of nucleotide bases, or two complimentary strands, which may form a double helix structure. "RNA" (ribonucleic acid) means any chain or sequence of the chemical building blocks adenine (A), guanine (G), cytosine (C) and uracil (U), called nucleotide bases that are linked together on a ribose sugar backbone. RNA typically has one strand of nucleotide bases, such as mRNA. Included are also single- and doublestranded hybrid molecules, i.e., DNA-DNA, DNA-RNA and RNA-RNA.

The nucleic acid molecule may also be modified by any means known in the art. Non-limiting examples of such modifications include methylation, substitution of one or more of the naturally occurring nucleotides with an analogue, and internucleotide modifications such as, for example, those with uncharged linkages (e.g., methyl phosphonates, phosphotriesters, phosphoramidates, carbamates, etc.) and with charged linkages (e.g., phosphorothioates, phosphorodithioates, etc.). Nucleic acid molecules, in the following also referred as polynucleotides, may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), alkylators, fluorophore (e.g. an Alexa or Cy dye), a fluorescent quencher or biotin. The polynucleotides may be derivatized by formation of a methyl or ethyl phosphotriester or an alkyl phosphoramidate linkage. Further included are nucleic acid mimicking molecules known in the art such as synthetic or semi-synthetic derivatives of DNA or RNA and mixed polymers.

Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1).

Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule considered according to the invention may additionally comprise promoters, enhancers, response elements, signal sequences, polyadenylation sequences, introns, 5'- and 3'- non-coding regions, and the like.

The term "protein" (also referred to as "polypeptide") as used herein interchangeably with the term "polypeptide" describes linear molecular chains of amino acids, including single chain proteins or their fragments, containing at least 50 amino acids. The term "peptide" as used herein describes a group of molecules consisting of up to 49 amino acids. The term "peptide" as used herein describes a group of molecules consisting with increased preference of at least 15 amino acids, at least 20 amino acids at least 25 amino acids, and at least 40 amino acids. The group of peptides and polypeptides are referred to together by using the term "(poly)peptide". (Poly)peptides may further form oligomers consisting of at least two identical or different molecules. The corresponding higher order structures of such multimers are, correspondingly, termed homo- or heterodimers, homo- or heterotrimers etc.. Furthermore, peptidomimetics of such proteins/(poly)peptides where amino acid(s) and/or peptide bond(s) have been replaced by functional analogues are also encompassed by the invention. Such functional analogues include all known amino acids other than the 20 gene-encoded amino acids, such as selenocysteine. The terms "(poly)peptide" and "protein" also refer to naturally modified (poly)peptides and proteins where the modification is effected e.g. by glycosylation, acetylation, phosphorylation, ADP-ribosylation and similar modifications which are well known in the art.

The enzyme being capable of cleaving the diphosphate linkage, triphosphate or tetraphosphate linkage is an enzyme being capable of decapping a 5'-dinucleotide-capped nucleic acid molecule by cleaving the diphosphate, triphosphate or tetraphosphate linkage that connects the two nucleotides of the dinucleotide that is attached to the 5' end of the nucleic acid sequence. Without wishing to be bound by this therapy it is beloved that the enzyme being capable of cleaving the diphosphate, triphosphate or tetraphosphate linkage within the dinucleotide cuts or preferentially cuts, such that one phosphate group remains linked to the nucleic acid molecule.

In accordance with the present invention this enzyme (a) comprises or consists of the amino acid sequence of SEQ ID NO: 21, (b) comprises or consists of an amino acid sequence being at least 80%, preferably at least 90% and most preferably at least 95% identical to SEQ ID NO: 21, (c) comprises or consists of the amino acid sequence being encoded by the nucleotide sequence of SEQ ID NO: 17, or (d) comprises or consists of an amino acid sequence being encoded by a nucleotide sequence being at least 80%, preferably at least 90% and most preferably at least 95% identical to SEQ ID NO: 17. The first aspect of the invention also covers (II) a nucleic acid molecule, preferably a vector, encoding in expressible form the enzyme of (I); and/or (III) a host cell comprising the nucleic acid molecule, preferably the vector of (II).

The amino acid sequence of the T4 phage Nudix hydrolase NudE.1 is SEQ ID NO: 21 and the encoding nucleotide sequence is SEQ ID NO: 17.

The amino acid sequence of the T4 phage Nudix hydrolase NudE.1 of SEQ ID NO: 21 comprises at the end a 6xHis-tag. This 6xHis-tag is advantageous for the purification of the enzyme but is not required for the enzymatic activity. If follows that in accordance with the present invention the enzyme being capable of cleaving the diphosphate linkage, triphosphate or tetraphosphate linkage may also (a') comprise or consist of the amino acid sequence of SEQ ID NO: 21 without the 6xHis-tag, or (b) comprise or consist of an amino acid sequence being at least 80%, preferably at least 90% and most preferably at least 95% identical to SEQ ID NO: 21 without the 6xHis-tag.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment for two or more sequences or subsequences the percentage of amino acid residues or nucleotides that are the same (e.g. 70%, 75%, 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 80% identical, preferably at least 85% identical, more preferably at least 90% identical, and most preferred at least 95% are envisaged by the invention. However, also envisaged by the invention are with increasing preference sequence identities of at least 97.5%, at least 98.5%, at least 99%, at least 99.5%, at least 99.8%, and 100%.

The term "vector" in accordance with the invention means preferably a plasmid, cosmid, virus, bacteriophage or another vector used e.g. conventionally in genetic engineering which carries the nucleic acid molecule of the invention. The nucleic acid molecule of the invention may, for example, be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1 neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

The nucleic acid molecule to be inserted into the vector can e.g. be synthesized by standard methods. Ligation of the coding sequences to transcriptional regulatory elements and/or to other amino acid encoding sequences can also be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators; see above), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide encoding the polypeptide/protein or fusion protein of the invention is operatively linked to such expression control sequences allowing expression in prokaryotes or eukaryotic cells. The vector may further comprise nucleic acid sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector comprises a selectable marker. Examples of selectable markers include genes encoding resistance to neomycin, ampicillin, hygromycine, chloramphenicol, and kanamycin. Specifically-designed vectors allow the shuttling of DNA between different hosts, such as bacteria-fungal cells or bacteria-animal cells (e. g. the Gateway system available at Invitrogen). An expression vector according to this invention is capable of directing the replication, and the expression, of the polynucleotide and encoded fusion protein of this invention. Apart from introduction via vectors such as phage vectors or viral vectors (e.g. adenoviral, retroviral), the nucleic acid molecules as described herein above may be designed for direct introduction or for introduction via liposomes into a cell. Additionally, baculoviral systems or systems based on vaccinia virus or Semliki Forest virus can be used as eukaryotic expression systems for the nucleic acid molecules of the invention.

The term "in expressible form" as used herein means that the nucleic acid molecule, preferably vector, comprises a nucleic acid sequence that encodes in the enzyme of (I) such that when the vector is introduced into a cell or cellular system the encoding nucleic acid sequence is expressed from the nucleic acid molecule, preferably vector as mRNA and translated from the mRNA into the enzyme of (I).

For instance, the nucleic acid sequence that encodes the enzyme of (I) may be comprised in the nucleic acid molecule, preferably vector in the form of an expression cassette. An expression cassette is a distinct component of a nucleic acid molecule, preferably vector comprising or consisting of a nucleic acid sequence that encodes in the enzyme of (I) and regulatory sequence(s) to be expressed by a host cell comprising in the nucleic acid molecule, preferably vector.

Hence, the nucleic acid molecule, preferably vector may then be introduced into a host cell and the host cell can be cultured under conditions wherein the enzyme of (I) is produced. The host cell comprising the nucleic acid molecule, preferably the vector of (II).

The term "host cell" means any cell of any organism that is selected, modified, transformed, grown, or used or manipulated in any way, for the production of the enzyme of (I) by the cell.

The host cell according invention is typically produced by introducing the nucleic acid molecule or vector of the invention into the host cell which upon its/their presence mediates the expression of the nucleic acid molecule encoding the enzyme of (I). The host from which the host cell is derived or isolated may be any prokaryote or eukaryotic cell or organism, preferably with the exception of human embryonic stem cells that have been derived directly by destruction of a human embryo.

Suitable prokaryotes (bacteria) useful as hosts for the invention are, for example, those generally used for cloning and/or expression like E. coli (e.g., E coli strains BL21, HB101, DH5a, XL1 Blue, Y1090 and JM101), Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis, Streptomyces coelicolor or Bacillus subtilis. Among this list E. coli is preferred. Appropriate culture mediums and conditions for the above-described host cells are well known in the art.

A suitable eukaryotic host cell may be a vertebrate cell, an insect cell, a fungal/yeast cell, a nematode cell or a plant cell. The fungal/yeast cell may a Saccharomyces cerevisiae cell, Pichia pastoris cell or an Aspergillus cell. Preferred examples for host cell to be genetically engineered with the nucleic acid molecule or the vector(s) of the invention is a cell of yeast, E. coli and/or a species of the genus Bacillus (e.g., B. subtilis). In one preferred embodiment the host cell is a yeast cell (e.g. S. cerevisiae).

In a different preferred embodiment, the host cell is a mammalian host cell, such as a Chinese Hamster Ovary (CHO) cell, mouse myeloma lymphoblastoid, human embryonic kidney cell (HEK-293), human embryonic retinal cell (Crucell's Per.C6), or human amniocyte cell (Glycotope and CEVEC). The cells are frequently used in the art to produce recombinant proteins. CHO cells are the most commonly used mammalian host cells for industrial production of recombinant protein therapeutics for humans.

The mammalian host cell is preferably an isolated host cell that does not form part of a mammal, preferably a human.

As can be taken from the appended examples, it was surprisingly found that NudE.1 from the phage T4 but not NudC from the host of T4 Escherichia coli is capable of hydrolyzing ADP-ribosylated rL2 proteins. Without wishing to be bound by this theory the difference between NudE.1 and NudC might be due to NudC's intrinsic RNA-binding affinity that may increase its activity on RNAylated protein compared to ADP-ribosylated protein. It is also of note that NudC is enzymatically active as a homodimer (see Höfer et al., Nat Chem Biol. 2016 Sep; 12(9): 730-734.) while NudE.1 is believed to act as monomer. In addition the examples show that NudE.1 from T4 is not only capable of hydrolyzing ADP-ribosylated protein. NudE.1 shows a broad substrate range on NAD-derived biomolecules including NAD, NAD-RNA but also related PTMs such as ADP-ribosylation and RNAylation.

The present invention relates in a second aspect to the use of an enzyme, nucleic acid molecule and/or a host cell for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein (I) the enzyme being capable of cleaving the diphosphate, triphosphate or tetraphosphate linkage, (a) comprises or consists of the amino acid sequence of SEQ ID NO: 21, (b) comprises or consists of an amino acid sequence being at least 80%, preferably at least 90% and most preferably at least 95% identical to SEQ ID NO: 21, (c) comprises or consists of the amino acid sequence being encoded by the nucleotide sequence of SEQ ID NO: 17, or (d) comprises or consists of an amino acid sequence being encoded by a nucleotide sequence being at least 80%, preferably at least 90% and most preferably at least 95% identical to SEQ ID NO: 17; (II) c and/or (III) the host cell comprises the nucleic acid molecule, preferably the vector of (II).

The present invention relates in a third aspect to a kit for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein the kit comprises (I) an enzyme being capable of cleaving the diphosphate, triphosphate or tetraphosphate linkage, wherein the enzyme (a) comprises or consists of the amino acid sequence of SEQ ID NO: 21, (b) comprises or consists of an amino acid sequence being at least 80%, preferably at least 90% and most preferably at least 95% identical to SEQ ID NO: 21, (c) comprises or consists of the amino acid sequence being encoded by the nucleotide sequence of SEQ ID NO: 17, or (d) comprises or consists of an amino acid sequence being encoded by a nucleotide sequence being at least 80%, preferably at least 90% and most preferably at least 95% identical to SEQ ID NO: 17; (II) a nucleic acid molecule, preferably a vector, encoding in expressible form the enzyme of (I); and/or (III) a host cell comprising the nucleic acid molecule, preferably the vector of (II).

The above definitions and preferred embodiments of the first aspect of the invention apply *mutatis mutandis* to the second and third aspect of the invention, as far as being amenable with the second and/or third aspect of the invention.

The kit of the third aspect of the invention implements a/the means required for conducting the method of the first or the use of the third aspect of the invention in the format of a kit.

The various components of the kit may be packaged into one or more containers such as one or more vials. The vials may, in addition to the components, comprise preservatives or buffers for storage. The kit may comprise instructions how to use the kit, which preferably inform how to use the components of the kit for for decapping a 5'-dinucleotide-capped nucleic acid molecule.

In accordance with a preferred embodiment of all aspects of the present invention the dinucleotide is 7-methyl-guanosine-5'-triphosphate-5'-guanosine, diadenosine tetraphosphate, NXD or FXD, wherein X is a nucleobase, preferably a purine or pyrimidine nucleobase, more preferably adenine, guanine, cytosine, thymine, or uracil and most preferably adenine.

NXD is a nicotinamide nucleobase dinucleotide (NAD) and FXD is a flavin nucleobase dinucleotide (FAD). The "X" nucleobase of the NXD or FXD can be a purine base or a pyrimidine base and is preferably selected from adenine, guanine, cytosine, thymine, and uracil.

Purine bases are preferred as compared to pyrimidine bases.

The five preferred nucleobase adenine (A), cytosine (C), guanine (G), thymine (T), and uracil (U) are called primary or canonical. They function as the fundamental units of the genetic code, with the bases A, G, C, and T being found in DNA while A, G, C, and U are found in RNA. Thymine and uracil are distinguished by the presence or absence, respectively, of a methyl group on the fifth carbon (C5) of these heterocyclic six-membered rings. Adenine and guanine have a fused-ring skeletal structure derived of purine, hence they are member of the class of purine bases. The ring structure of cytosine, uracil, and thymine is derived of pyrimidine, so that they are members of the class of pyrimidine bases.

Among the five preferred nucleobase adenine (A), cytosine (C), guanine (G), thymine (T), and uracil (U) adenine (A) is preferred in view of the appended examples with NAD.

Further nucleobases are, for example, xanthine, hypoxanthine, 7-methylguanine, 2,6-diaminopurine, and 6,8-diaminopurine (purine bases), pseudouridine, N1-methyl-pseudouridine or 5,6-dihydrouracil, 5-methyluracil and 5-hydroxymethylcytosins (pyrimidine base). Among this list of further nucleobases 7-methylguanine is preferred because it is illustrated by the appended examples.

NXD and FXD are most preferably NAD and FAD.

Nicotinamide adenine dinucleotide (NAD) is a coenzyme central to metabolism. Found in all living cells, NAD is called a dinucleotide because it consists of two nucleotides joined through their two phosphate groups. One nucleotide contains an adenine nucleobase and the other nicotinamide. NAD exists in two forms: an oxidized and reduced form, abbreviated as NAD+ and NADH (H for hydrogen), respectively. Flavin adenine dinucleotide (FAD) is a redox-active coenzyme associated with various proteins, which is involved with several enzymatic reactions in metabolism. A flavoprotein is a protein that contains a flavin group, which may be in the form of FAD or flavin mononucleotide (FMN). Many flavoproteins are known: components of the succinate dehydrogenase complex, α-ketoglutarate dehydrogenase, and a component of the pyruvate dehydrogenase complex.

In accordance with a preferred embodiment of all aspects of the present invention, the dinucleotide-capped nucleic acid molecule is a dinucleotide-capped RNA molecule.

While it is believed that the method, use and kit of the invention also work with dinucleotide-capped DNA molecules, in particular single-stranded dinucleotide-capped DNA molecules dinucleotide-capped RNA molecules are preferred in view of the appended examples. As explained above RNA is generally and also preferred single-stranded.

In accordance with another preferred embodiment of all aspects of the present invention, the enzyme being capable of cleaving the diphosphate, triphosphate or tetraphosphate linkage comprises the sequence GXXXXXEXXXXXUAXREXXEEXGU (SEQ ID NO: 25), wherein X can be any amino acid and U is a bulky, hydrophobic amino acid, preferably Phe, Ile, Leu, or Val.

In accordance with a more preferred embodiment of all aspects of the present invention, the enzyme being capable of cleaving the diphosphate, triphosphate or tetraphosphate linkage comprises the sequence GRVENSDLSALDAARRECLEETGF (SEQ ID NO: 26) or a sequence being at least 80%, preferably at least 90% and most preferably at least 95% identical thereto, provided that the underlined amino acids are retained.

NudE.1 is member of the Nudix hydrolase superfamily that consists of widely distributed subfamilies of enzymes characterized by the shared structural motif of SEQ ID NO: 25; see Lu et al. (2002), THE JOURNAL OF BIOLOGICAL CHEMISTRY, 277(26):23181-23185. In NudE.1 shared structural motif has SEQ ID NO: 26.

Enzymological and structural studies have shown that the arrangement of amino acids in the shared structural motif leads to a loop-helix-loop motif being involved in substrate binding and catalysis.

In accordance with a preferred embodiment the present invention, in the method or use the decapping is carried out in or the kit further comprises 0.5 mM to 2mM DTT, preferably about 1 mM DTT and/or a degradation buffer, wherein the degradation buffer comprises one or more and preferably all of 10 to 50 mM, preferably about 25 mM Tris-HCl pH 7.5,
25 to 100 mM NaCl, preferably about 50 mM NaCl,
25 to 100 mM KCI, preferably about 50 mM KCI, and
2.5 to 20 MgCl₂, preferably about 10 mM MgCl₂.

The above experimental conditions closely resemble the experimental conditions as used in the appended examples. The term "about" as used herein means with increasing preference ±20%, ±10%, and ±5%. The "one or more" are preferably two or more and more preferably three or more.

While Dithiothreitol (DTT) is not mandatory it was found that DTT increases the efficiency of the decapping. DTT has the C₄H₁₀O₂S₂. DTT is a reducing agent; once oxidized, it forms a stable six-membered ring with an internal disulfide bond. It has a redox potential of -0.33 V at pH 7. DTT is frequently used to reduce the disulfide bonds of proteins and, more generally, to prevent intramolecular and intermolecular disulfide bonds from forming between cysteine residues of proteins.

In the examples the following buffer was used:
25 mM Tris-HCl, pH 7.5
50 mM NaCl
50 mM KCI
10 mM MgCl2
1 mM DTT

Also a buffer with only 50% of the molar concentration was tested and found to work:
12.5 mM Tris-HCl, pH 7.5
25 mM NaCl
25 mM KCI
5 mM MgCl2
1 mM DTT

With regard to MgCl₂ it is noted that it is expected that it can be replaced by other salts of divalent cations, in particular by MnCl₂.

In accordance with a preferred embodiment of the first and second aspect of the present invention the method or use further comprises the degradation of the uncapped nucleic acid molecule by a RNase and/or DNase.

In accordance with a preferred embodiment of the first and second aspect of the present invention the kit further comprises RNase and/or DNase for degrading the uncapped nucleic acid molecule.

The 5'-dinucleotide-cap protects nucleic acid molecules from degradation by RNase and/or DNase. If the cap is removed by the method or use of the invention the uncapped nucleic acid molecule can be degraded by RNase and/or DNase.

In accordance with a preferred embodiment of the first and second aspect of the present invention the method or use further comprises prior to the decapping attaching a dinucleotide to the 5'-end of a nucleic acid molecule.

Means and method for attaching a dinucleotide to the 5'-end of a nucleic acid molecule are known, for example, from Huang F. Efficient incorporation of CoA, NAD and FAD into RNA by in vitro transcription. Nucleic Acids Res. 1;31(3):e8 (2003). or Höfer K, Abele F, Schlotthauer J, Jäschke A. Synthesis of 5'-NAD-Capped RNA. Bioconjug Chem. 20;27(4):874-7 (2016).

In accordance with another preferred embodiment of the first and second aspect the method or use further comprises prior to the decapping isolating a 5'-dinucleotide-capped nucleic acid molecule from a total RNA sample, preferably by NAD captureSeq, NADcapPro or CapzymeSeq.

Means and methods for isolating a 5'-dinucleotide-capped nucleic acid molecule from a total RNA sample are known. Preferred but non-limiting examples are NAD captureSeq, NADcapPro or CapzymeSeq.

NAD captureSeq is used in the appended examples and is described in Winz, M. L. et al. Capture and sequencing of NAD-capped RNA sequences with NAD captureSeq. Nat Protoc 12, 122-149 (2017).

NADcapPro is described in Sharma et al., NADcapPro and circNC: methods for accurate profiling of NAD and non-canonical RNA caps in eukaryotes, Communications Biology, 6:406 (2023).

CapzymeSeq is described in Vvedenskaya et al., CapZyme-Seq Comprehensively Defines Promoter-Sequence Determinants for RNA 5' Capping with NAD+, Molecular Cell, 70(3):553-564.e9.

In accordance with a more preferred embodiment of all aspects of the present invention the 5'-dinucleotide-capped nucleic acid molecule is covalently attached to a protein or peptide.

Means and methods for covalently attaching a protein or peptide to the 5'-dinucleotide-capped nucleic acid molecule are known. In particular ADP-ribosyltransferases can be used in order to attach a 5'-NXD-capped nucleic acid molecule to a protein or peptide; see WO 2023/006264.

In WO 2023/006264 it is shown that the bacteriophage T4 ARTs accept not only NAD, but also NXD-nuclei acid molecules as substrate, thereby covalently linking entire RNA chains to acceptor proteins in an "RNAylation" reaction. It is shown that ARTs also work with NGD (5'-nicotinamidguanindinucleotide)-RNA, NCD (5'-nicotinamidcytosindinucleotide)-RNA and (5'-nicotinamiduracildinucleotide) NUD-RNA.

In accordance with a more preferred embodiment of the first and second aspect prior to the decapping the 5'-dinucleotide-capped nucleic acid molecule is attached to the protein or peptide by an ADP-ribosyltransferase (ART), wherein the ART preferably comprises or consists of SEQ ID NO: 27 or SEQ ID NO: 27 or a sequence being at least 80% identical thereto.

In accordance with a related more preferred embodiment of the third aspect the kit further comprises an ART for attaching the 5'-dinucleotide-capped nucleic acid molecule to the protein or peptide, wherein the ART preferably comprises or consists of SEQ ID NO: 27 or SEQ ID NO: 28 or a sequence being at least 80% identical thereto.

SEQ ID NO: 27 is the amino acid sequence of the ART ModB from *Escherichia* virus T4 as deposited under Acc. No. CAA67254.1. SEQ ID NO: 28 is the amino acid sequence of the cloned ModB as used in WO 2023/006264 which comprises in addition a His6 tag that may serve for the purification of ModB, for example, after it has been recombinantly produced from an expression vector as described herein below for the heterologous fusion protein.

In accordance with a more preferred embodiment of all aspects of the present invention the 5'-dinucleotide-capped nucleic acid molecule is covalently attached at its 3'-end to N-acetylgalactosamine (GaINAc), preferably through a bivalent or trivalent branched linker.

N-acetylgalactosamine (GalNAc) is an amino sugar derivative of galactose. GalNAc is used as a targeting ligand in investigational antisense oligonucleotides and siRNA therapies targeted to the liver, where it binds to the asialoglycoprotein receptors on hepatocytes; see, for example, Nair, et al. (2014), Journal of the American Chemical Society, 136(49):16958-16961. For the purpose hepatocyte targeting GalNAc can also be used in the present application.

By a bivalent or trivalent branched linker two or three copies can be attached to the same 3'-end of a nucleic acid molecule. The attachment of GalNAc to nucleic acid molecules and suitable bivalent or trivalent branched linker are described, for example, in WO 2012/177784.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

The figures show.

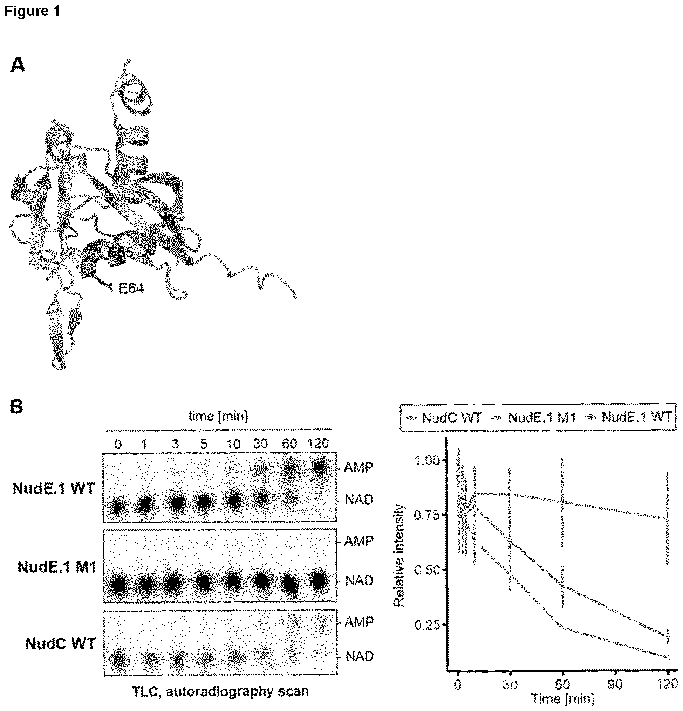

### Figure 1: T4 phage Nudix hydrolase NudE.1 hydrolyzes NAD in vitro.

A) Alphafold prediction for NudE.1 indicating E64 and E65 whose mutation to glutamines inactivates NudE.1 (M1). B) Activity of NudE.1 WT, NudE.1 M1 (E64,65Q) and NudC WT on radiolabeled ³²P-NAD *in vitro* as monitored by thin-layer chromatography (TLC) and autoradiography. NudE.1 and NudC WT hydrolyze NAD giving rise to AMP and NMN (n=3).

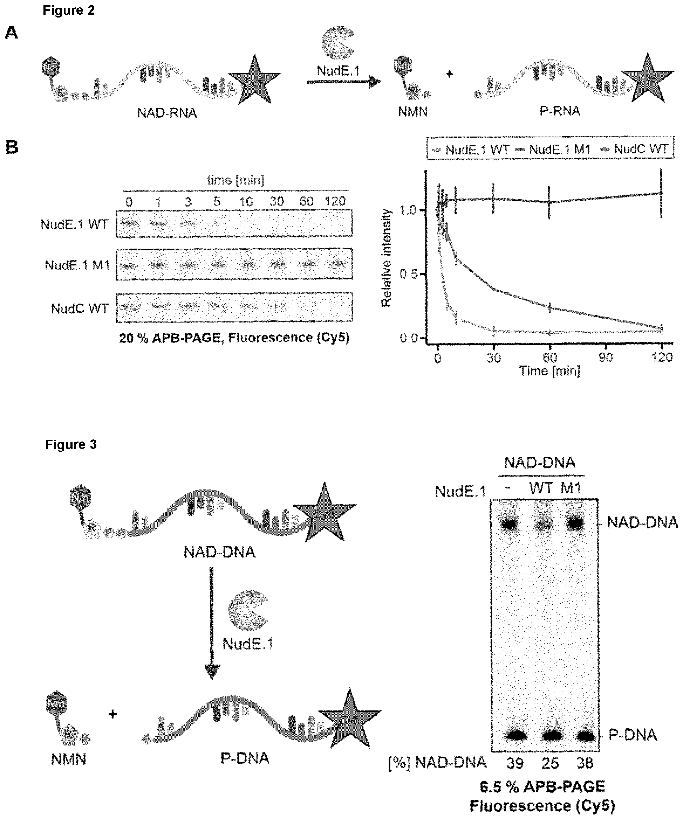

### Figure 2: Hydrolysis of NAD-capped RNA by the T4 phage Nudix hydrolase NudE.1 in vitro.

**A**) Reaction schematic for NudE.1-mediated hydrolysis of the NAD-cap/NAD-RNA decapping using a 3'-Cy5-labelled NAD-RNA as substrate. Upon hydrolysis, nicotinamide mononucleotide (NMN) is liberated and the NAD-RNA is converted in a 5'-monophosphorylated RNA (P-RNA). **B**) 20 % acrylamidophenyl boronic acid polyacrylamide gel electrophoresis (APB-PAGE) analysis of NAD-RNA decapping by NudE.1 WT, its inactive mutant NudE.1 M1 (E64,65Q mutation) and E. *coli* Nudix hydrolase NudC (left panel). 500 nM RNA substrate were incubated in the presence of 25 nM enzyme at 37 °C. NAD-RNA was visualized by the 3'-Cy5-label. NudE.1 decaps NAD-RNA more efficiently than NudC as demonstrated by the relative intensity of the NAD-RNA throughout the time course of the NAD-cap hydrolysis reactions (right panel, n=3 biologically independent replicates).

### Figure 3: Hydrolysis of NAD-capped DNA by the T4 phage Nudix hydrolase NudE.1 in vitro.

Reaction schematic for NudE.1-mediated hydrolysis of the NAD-cap/NAD-DNA decapping using a 3'-Cy5-labelled NAD-DNA as substrate. Upon hydrolysis, nicotinamide mononucleotide (NMN) is liberated and the NAD-DNA is converted in a 5'-monophosphorylated DNA (P-DNA) (left panel). 6.5 % APB-PAGE analysis of NAD-DNA decapping by NudE.1 WT, its inactive mutant NudE.1 M1 or in the absence of enzyme (right panel). 250 nM DNA substrate were incubated in the presence of 100 nM enzyme at 37 °C for 60 min. NAD-DNA was visualized using the 3'-Cy5-label. NudE.1 is able to decap NAD-DNA shown by decrease in the NAD-DNA fraction (right panel, n=1 biologically independent replicates).

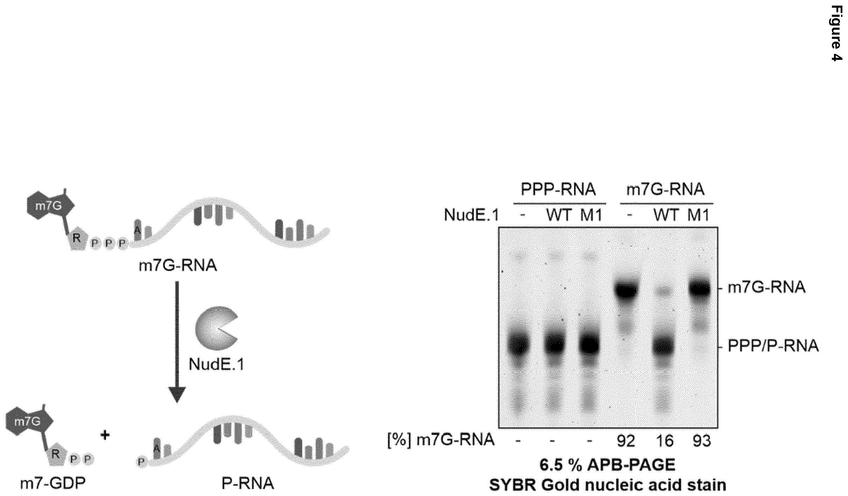

### Figure 4: Hydrolysis of m7G-capped RNA by the T4 phage Nudix hydrolase NudE.1 in vitro.

Reaction schematic for NudE.1-mediated hydrolysis of the m7G-cap/m7G-RNA decapping using a 108 nt m7G-capped RNA (*E. coli* RNAI) as substrate. Upon hydrolysis, 7-methylated guanosine diphosphate (m7-GDP) is released and the m7G-RNA is converted to a 5'-monophosphorylated RNA (P-RNA) (left panel). 6.5 % APB-PAGE analysis of m7G-RNA decapping by NudE.1 WT, its inactive mutant NudE.1 M1 or in the absence of enzyme (right panel). 250 nM DNA substrate were incubated in the presence of 100 nM enzyme at 37 °C for 60 min. m7G-RNA was visualized using the 3'-Cy5-label. NudE.1 is able to decap m7G-RNA shown by decrease in the m7G-RNA fraction (right panel, n=1 biologically independent replicates). 5'-triphosphorylated (PPP-RNA) and P-RNA remain stable in the presence of NudE.1 indicating the absence of nuclease activity of NudE.1.

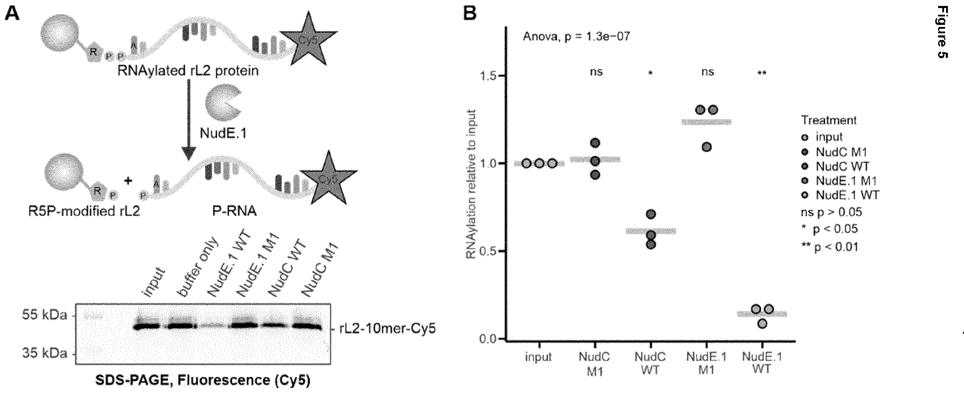

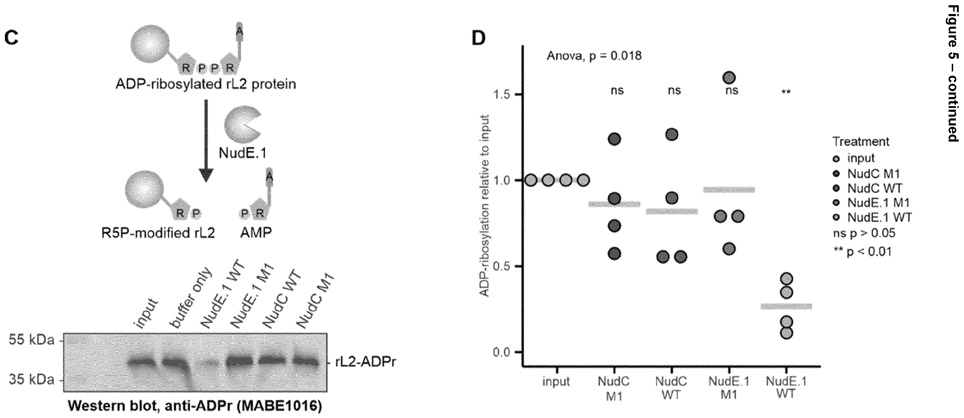

### Figure 5: Processing of ADP-ribosylation and RNAylation by NudE.1 in vitro.

**A**) Schematic removal of the RNAylation of rL2 by NudE.1 WT giving rise to a ribose-5-phosphate (R5P)-modified protein (top panel). 10 % SDS-PAGE analysis of the removal of the RNAylation from RNAylated protein rL2 (rL2-10mer-Cy5) by Nudix hydrolases represented by loss of Cy5 signal (bottom panel, n=3).
**B**) Quantification of rL2 RNAylation after treatment with Nudix hydrolases. RNAylation of rL2 is significantly reduced in the presence of NudE.1 WT and NudC WT (n=3). Statistical tests were performed as two-sided t-tests at a significance level of 0.05 using ggpubr. C) Schematic removal of the ADP-ribosylation of rL2 by NudE.1 WT giving rise to a ribose-5-phosphate (R5P)-modified protein (top panel). Western blot analysis of the removal of the ADP-ribosylation from ADP-ribosylated rL2 protein (rL2-ADPr) by Nudix hydrolases represented by loss of anti-ADPr (MABE1016) signal (bottom panel, n=4). D) Quantification of rL2 ADP-ribosylation after treatment with Nudix hydrolases. ADP-ribosylation of rL2 is significantly reduced in the presence of NudE.1 WT and NudC WT (n=4). Statistical tests were performed as two-sided t-tests at a significance level of 0.05 using ggpubr.

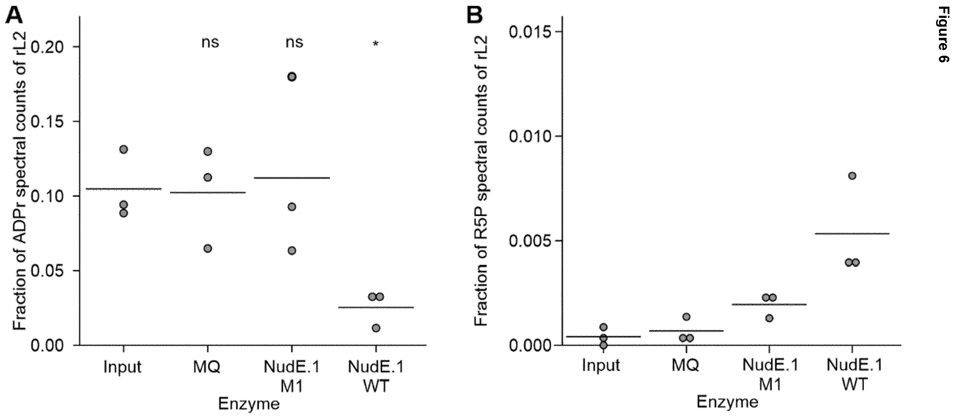

### Figure 6: Proteomic analysis of processing of rL2 ADP-ribosylation by NudE.1 in vitro.

Spectral counts of ADP-ribosylated (ADPr, **A**) or R5P-modified (**B**) rL2 peptides relative to all spectral counts for rL2 derived from proteomic analysis in biological triplicates (n=3). Input represents untreated, ADP-ribosylated rL2, whilst the other options present treated samples including mock-treated (MQ), NudE.1 M1 and NudE.1 WT. Significance tests in A were performed as two-sided t-tests against Input as reference at a significance level of 0.05 using ggpubr. Adjusted p-value was found significant (0.04, *) comparing Input and NudE.1 WT sample only.

The examples illustrate the invention.

### Example 1 - Results

### The phage Nudix hydrolase NudE.1 hydrolyses NAD- and m7G-caps

In *E. coli,* degradation of NAD-RNAs is initiated by NAD-cap hydrolysis by the Nudix hydrolase NudC - referred to as decapping here ^{1,2}. It was wondered whether additional Nudix hydrolases may play roles in NAD-RNA decapping during T4 phage infection. The T4 phage actually encodes one Nudix hydrolase - called NudE.1 - that has been described to hydrolyze adenosine-derived cofactors and metabolites, primarily ADP-ribose, Ap₃A and FAD, within their diphosphate moiety ³. In this study in 2002, no activity of NudE.1 on NAD was reported ³.

AlphaFold2 ⁴ was used to predict the structure of NudE.1, which reveals a wide-open cleft in which the catalytically relevant Nudix motif is located (Figure 1A). Based on this potential active site conformation, was speculated that NudE.1 may also except substrates such as NAD and NAD-RNA for hydrolysis. It expressed and purified NudE.1 migrating as an apparent monomer during size exclusion chromatography (SEC). Initially, the activity of NudE.1 was assessed on NAD using site-specifically ³²P-labelled NAD. It was observed that NudE.1 efficiently hydrolyses NAD into nicotinamide mononucleotide (NMN) and AMP, whilst a mutant of the catalytic site E64,65Q (NudE.1 M1) did not exert hydrolysis activity on NAD (Figure 1B).

Next, the potential NAD-RNA decapping activity was tested a 10-nucleotide NAD-RNA with a 3'-Cy5-label (NAD-10mer-Cy5) (Figure 2A). Similar to the known decapping enzyme NudC, NudE.1 efficiently decapped NAD-RNA *in vitro,* whilst the inactive mutant showed no measurable decapping activity (Figure 2B). To assess whether NudE.1 prefers certain RNA structure for decapping. Our analysis revealed no clear preference for the structure of the 5'-ends.

Further, it was assessed whether NudE.1 could exert the same NAD-cap hydrolysis activity, when the capped nucleic acid is not RNA but DNA (Figure 3). Therefore, NAD-capped was generated DNA with a 3'-Cy5-label using imidazolide chemistry ⁵. Indeed, a reduction of the NAD-DNA decapping by NudE.1 was observed, whilst the inactive mutant did not reduce the NAD-DNA fraction (Figure 3).

The NAD-cap serves as a representative of "non-canonical" cap structures in all domains of life ². In the eukaryotic domain of life however, mRNA molecules are "canonically" capped with 7-methyl-guanosine (m7G) that basically forms a dinucleotide with the 5'-nucleotide of the RNA via a triphosphate bond ⁶. Given the similar natures of the NAD- and m7G-cap as dinucleotide structures it was speculated that NudE.1 may also cleave within the triphosphate bond within the cap of m7G-capped RNA. Indeed, a strong reduction of the m7G-capped RNA fraction was observed yielding decapped 5'-P-RNA only in the presence of NudE.1 WT (Figure 4).

### NudE.1 processes the post-translational proteinmodifcations ADP-ribosylation and RNAylation

NudE.1 was reported to specifically exert activity on ADP-ribose, FAD and Ap3A *in vitro* ³. Here, however, hydrolysis activity on NAD and NAD-capped RNA, DNA as well as m7G-capped RNA was also recorded *in vitro.* Based on this observation and the predicted structure with a wide-open catalytic site (Figure 1A), it was speculated that NudE.1 could also process NAD- and NAD-RNA-derived post-translational protein modifications. Recently, the T4 phage-encoded ADP-ribosyltransferase ModB was reported to use NAD to ADP-ribosylate or NAD-RNA to RNAylate *E. coli* proteins at arginine residues ⁷. Thereby, ModB creates specific conjugates of proteins of the translational apparatus and ADP-ribose or ADP-ribose-RNA, respectively, in order to regulate cellular processes such as translation ⁷. It remains unknown, however, whether there are ways for the phage to reverse these modifications.

Therefore, RNAylated and ADP-ribosylated rL2 proteins were generated with ModB *in vitro* and subjected them to treatment by NudE.1 and NudC. A significant reduction of both ADP-ribosylation and RNAylation signals was observed in the presence of NudE.1 WT only, whilst its catalytically inactive mutant did not remove these PTMs (Figure 5 A-D).

It was supposed that due to the catalytic target site of Nudix hydrolases - the pyrophosphate moiety - Nudix-mediated hydrolysis of ADP-ribosylation and RNAylation may give rise to AMP or 5'-P-A-RNA, respectively. This would result in a ribose-5-phosphate (R5P) residue left at the formerly ADP-ribosylated or RNAylated protein (Figure 5A,C). Therefore, proteomics was employed in order to determine overall ADP-ribose and R5P occupancy on ADP-ribosylated ribosomal protein L2 (rL2) as reported before ⁷ in the presence and absence of NudE.1. Upon incubation of ADP-ribosylated rL2 with NudE.1 WT, a significant reduction of ADP-ribosylation of rL2 was recorded as demonstrated by reduced fractional spectral counts of ADP-ribosylated rL2 peptides (Figure 6A). In the same line, R5P-containing peptide spectra rose in abundance upon NudE.1 WT treatment (Figure 6B) confirming the proposed mode of action, where NudE.1 hydrolyzes the diphosphate within the ADP-ribose moiety. Accordingly, the same mode of action is proposed for the cleavage of RNAylated protein.

Altogether, our characterization of NudE.1 shows its broad substrate range on NAD-derived biomolecules including NAD, NAD-RNA but also related PTMs such as ADP-ribosylation and RNAylation, which play a role during T4 phage infection of E. *coli.* Moreover, it was demonstrated that NudE.1 is a versatile enzyme that may be used in decapping of both DNA and RNA molecules that are not only NAD-capped but also m7G-capped.

### Example 2 - Material & Methods

### General

All DNA and RNA oligos were purchased from Integrated DNA Technologies. Chemical compounds were obtained from Sigma Aldrich or Carl Roth GmbH, if not indicated otherwise. RNA was precipitated in the presence of 0.3 M NaOAc pH 5.5 and 1 volume isopropanol by centrifugation at 21,000 × g or 18,500 × g, 4 °C for 90 min after incubation at - 20 °C overnight, if not indicated differently.

### Preparation and characterization of NAD-RNAs by in vitro transcription

### Amplification of Qβ and RNAI dsDNA templates

PCR of Qβ and RNAI dsDNA template for *in vitro* transcription was performed using 50 nM fwd and 50 nM rev ultramer (Table 2) in the presence of 1 × GC buffer, 5 % DMSO, 0.1 mM of dNTP mix, 500 nM fwd primer, 500 nM rev primer and 0.01 U/µl Phusion polymerase. The PCR products were analyzed by 2 % agarose gel electrophoresis and purified using the Qiagen PCR purification kit. DNA concentrations were measured using the NanoDrop.

### IVT transcription of 5'-PPP-RNA and 5'-NAD-RNA

*In vitro* transcription of 5'-triphosphate (PPP) Qβ-RNA and RNAI was performed in a 20 µl scale using the HighYield T7 RNA Synthesis Kit (Jena Bioscience) according to manufacturer's instructions in the presence of 1 µg dsDNA template. NAD-capped RNA was synthesised using the same conditions except for the presence of 3.75 mM ATP and 7.5 mM NAD. The IVT reactions were incubated at 37 °C for 3 h. Residual DNA was removed by DNase I digestion using 20 U DNase I and 1 × DNase I incubation buffer and incubating the mixture at 37 °C for 30 min. *In vitro* transcribed 5'-PPP-RNAs and 5'-NAD-RNAs were purified by 10 % preparative PAGE. RNA bands were visualized by UV shadowing. The bands were excised, and RNA was eluted from the gel material by incubation in 4 ml 0.3 M NaOAc pH 5.5 by shaking at 600 rpm, 14 °C overnight. An additional elution step was performed for 3 h at the same settings. After removal of gel pieces, RNA was isopropanol precipitated, RNA pellets were air-dried and resuspended in 200 µl Millipore water. And then isopropanol precipitated again and resuspended in a final volume of 50 µl MQ water. RNA concentrations were measured on the NanoDrop. NAD-RNAs were analyzed using 10 % PAGE. The presence of the NAD-cap was confirmed by 6 % APB-PAGE (acrylamidophenyl boronic acid polyacrylamide gel electrophoresis ⁸) and NudC digest. Reactions were prepared for analysis in a 10 µl scale by incubating 100 ng of each RNA, 1 × degradation buffer and 4.2 µM of NudC (1 µl) or 1 µl Millipore water, respectively. The reactions were incubated at 37 °C, 30 min and applied to 6 % (w.r.t. final acrylamide concentration) APB-PAGE. Gels were imaged at ChemiDoc after staining in SYBR Gold (Invitrogen).

### m7G-capping of in vitro transcribed PPP-RNA

14.35 µg PPP-RNAI were m7G-capped (cap 0) using ScriptCap m7G Capping System (Cellscript) according to manufacturer's instructions and then purified using RNA Clean & Concentrator Kit (RCC-5, Zymo).

### NAD-capping of purchased RNA and DNA in vitro

Capping reactions were performed in a 50 µl scale using 40 µM 5'-P-RNA-Cy5, 40 µM 5'-P-DNA-Cy5 or 160 µM 5'-P-8mer (Table 3), 50 mM MgCl₂ and a tip of a spatula of imidazolide nicotinamide mononucleotide (Im-NMN) as previously described ⁵. Reactions were purified by centrifugation at 14,000 × g, 4 °C in 0.5 ml 3 kDa Amicon filters and washing with four column volumes of Millipore water. RNAs were eluted in a final volume of 50 µl and stored at - 20 °C. RNA concentrations and NAD-capping efficiencies were determined by 20 % PAGE or 20 % APB-PAGE analysis using unmodified input RNA as a reference and ImageLab 6.1 for quantification of band intensities. NAD-capping efficiencies accounted for around 50 % each.

### Synthesis of ³²P-labelled NAD

500 µM nicotinamide mononucleotide (NMN), 10 mM DTT, 330 nM alpha-³²P-ATP, 1 × degradation buffer and 2.14 µM NadR were incubated at 37 °C for 1 h. The reaction was purified by P/C/I extraction and monitored by by thin layer chromatography (TLC) using a 60:40 mixture of 100 % ethanol and 1 M NH4OAc pH 5 as mobile and Alugram TLC plates (Macherey Nagel) as stationary phase.

### Cloning of vectors for protein expression

The gene for NudE.1 (GeneID: 1258692; UniprotlD: P32271, Suplementary Table S2) was amplified from T4 phage solution by high-fidelity PCR using forward and reverse primers introducing sites for restriction-based cloning as indicated in Table 2. PCR products, purified via QIAQuick PCR purification kit (Qiagen), and pET28a vector, purified va GeneJet Plasmid Mini Prep Kit (Thermo Fisher), were both digested with Fast Digest Ncol and Xhol (both Thermo Fisher) and purified via PCR purification or gel extraction kit (both Qiagen). Vector and insert were ligated using T4 DNA ligase according to manufacturer's instructions and ligation products transformed into chemically competent *E. coli* DH5 alpha cells. Plasmids isolated from clones were validated for correct insert via Sanger sequencing (Microsynth Seqlab) and finally transformed into chemically competent *E. coli* BL21 DE3 cells for protein expression (Table 4).

A similar workflow was employed for site-directed mutagenesis of plasmids replacing the restriction-based approach with a primer-driven site-specific mutagenesis approach with 5'-monophosphorylated primers by PCR amplification and re-ligation of the PCR product with mutation. The plasmids containing the mutations in the insert were retrieved as described above.

### Expression and purification of proteins

Proteins were expressed from the respective plasmid (Tables 1 and 4) in *E. coli* BL21 DE3. Cells were grown in LB medium at 37 °C, 180 rpm to an OD₆₀₀ of 0.8, when protein expression was induced by addition of Isopropyl-beta-D-thiogalactoside to a final concentration of 1 mM. Bacteria were pelleted after incubation at 37 °C for 3 h. Pelleted bacteria were resuspended in Ni-NTA buffer A (50 mM Tris-HCl, pH 7.5, 1 M NaCl, 1 M urea, 5 mM MgSO4, 5 mM β-mercaptoethanol, 5% glycerol, 5 mM imidazole, one tablet complete EDTA-free protease inhibitor cocktail (Roche) per 1 I) and lysed by sonication (2 × 5 min at 80 % amplitude, 0.5 s pulse). The lysate was was cleared by ultra-centrifugation at 37,000 xg, 30 min, 4 °C and the supernatant was filtered through 0.45 µm filters. Proteins were purified from the supernatant by Ni-NTA affinity chromatography using either Ni-NTA agarose beads (Jena Bioscience, for gravity-based purification) or 1 ml Ni-NTA HisTrap column (GE Healthcare, for fast performance liquid chromatography (FPLC)-based purification). Proteins were eluted either with Ni-NTA buffer B (with 300 mM imidazole added, gravity-based) or a gradient using Ni-NTA buffer B (FPLC-based) and analysed by SDS-PAGE. An NGC system (Bio-Rad) was used for all FPLC-based protein purifications. Proteins were further purified by size-exclusion chromatography (SEC) using a Superdex 200 10/300 GL column (GE Healthcare) integrated in the NGC system. SEC buffer containing 300 mM NaCl and 50 mM Tris-HCl, pH 7.5 was used as running buffer. Fractions of interest were analysed by SDS-PAGE, pooled and concentrated in Amicon Ultra-4 centrifugal filters (molecular weight cut-off (MWCO) 10 kDa with centrifugation at 5,000 × g, 4 °C). Protein concentration was measured with a NanoDrop ND-1000 spectro- photometer (Thermo Fisher). Finally, proteins were stored in SEC buffer supplemented with 50% glycerol at -20 °C. For the estimation of the oligomeric state of the proteins, 150 µl protein analysed on a Superdex 200 10/300 GL column (GE Healthcare) integrated in an NGC system via constant flow of SEC buffer with known monomeric proteins serving as calibration standards. A custom Python Script was compiled to perform linear regression of calibration standards and estimate the oligomeric state of analysed proteins.

### AlphaFold2 prediction of NudE.1

The protein sequences of cloned NudE.1 WT and M1 (Table 3) were used to predict structures via the Google Colab Tool of AlphaFold2 - named ColabFold (version 1.5.2, access date 20^{th} June 2023) ⁴. Standard settings were applied expecting NudE.1 to occur as a monomer. Prediction from rank 1 was further analysed each in PyMol (version 2.4.1).

### In vitro decapping and hydrolysis assays

500 nM NAD-capped RNA-Cy5 (Table 3) were incubated in the presence of 1 mM DTT, 1 × degradation buffer (25 mM Tris-HCl pH 7.5, 50 mM NaCl, 50 mM KCI, 10 mM MgCl₂) and either 25 nM NudE.1 WT, M1 (E64,65Q) or NudC WT, M1 (E178Q). For NAD spike-in kinetics, NAD was included in the reaction at final concentrations of 350 µM (700-fold molar excess over NAD-RNA) or 750 µM (1,500-fold molar excess over NAD-RNA) and a spike-in of radioactive ³²P-NAD (if indicated, 2.6 nM). Reactions were incubated at 37 °C and samples taken throughout time-course of the reaction were immediately stopped by addition of 1 volume 2 × APB loading dye (8.3 M urea, 0.05 % (w/v) bromophenol blue, 0.05 % (w/v) xylene cyanol). A sample was taken before addition of enzyme each. Samples were analyzed by 20 % APB-PAGE and gels were imaged at ChemiDoc (Bio-Rad) using the Cy5 channel. Reactions using ³²P-NAD were stopped by heating samples at 90 °C for 1 min and were subsequently analysed by TLC. TLC plates were imaged by autoradiography using a Typhoon Imager (Amersham Biosciences). Intensity-based band quantification was performed in Image Lab (Bio-Rad).

NAD-DNA-Cy5 and m7G-RNAI decapping assays were performed under similar settings as described above with the following abberations. 100 nM DNA or RNA substrate were incubated in the presence of 250 nM NudE.1 WT or M1.

NAD only hydrolysis assays were performed using the same settings with the following aberrations. A final concentration of 25 µM NAD and a spike-in of radioactive ³²P-NAD (2.6 nM) were used instead of NAD-RNA-Cy5 in the presence of 1 µM NudE.1 WT, M1 or NudC WT. Reactions were analysed by TLC and autoradiography as described above.

### RNAylation and ADP-ribosylation of proteins and removal by Nudix hydrolases

6.5 µM rL2 were modified in the presence of 1 × transferase buffer (10 mM Mg(OAc)₂, 22 mM NH₄Cl, 50 mM Tris-acetate pH 7.5, 1 mM EDTA, 10 mM β-mercaptoethanol and 1 % glycerol ), 0.05 mg/ml ModB, and either 2.22 µM NAD-10mer-Cy5 (RNAylation) or 22.2 µM NAD at 15 °C for 90 min. Modified proteins were rebuffered in 1 × degradation buffer using Zeba columns (7 kDa molecular weight cut-off (7K MWCO)) according to manufacturer's instructions. 3.03 µM rebuffered rL2 were subsequently incubated with 1 mM DTT and 1 µM of either NudE.1 WT/M1 or NudC WT/M1 at 37 °C for 1 h. Removal of the RNAylation was monitored by 10 % SDS-PAGE following Cy5 imaging and stain-free imaging with TCE with the ChemiDoc imager (Bio-Rad). For analysis of the removal of the ADP-ribosylation, proteins were blotted to PVDF membranes as described ⁷. Protein was detected via stain-free imaging before blotting and ADP-ribosylation was detected with pan-ADPr-binding reagent (MABE1016) and visualized via Chemiluminescence with the ChemiDoc imager (Bio-Rad).

Modification signals and protein intensities were quantified with ImageLab. A custom R Script was used to normalize modification signals to protein intensities and normalized intensities from biological triplicates.

### Proteomics

Hydrolysis reactions of RNAylated and ADP-ribosylated rL2 in the presence of NudE.1 and NudC (WT/M1) as described above were digested with Trypsin (1:10 (w/w)) at 37 °C for 3 h. Peptides were C18 purified and analyzed via proteomics as described previously ⁷.

Spectral counts for ADP-ribosylated, R5P-modified and unmodified peptides were retrieved from Scaffold5 Software (version 5.0.1). ADP-ribose (ADPr) and ribose-5-phosphate (R5P) modifications were analysed relative to all spectral counts for rL2 protein using a custom R Script.

**Table 1: List of bacteria and phages used in this study.**

| **Strain** | **Purpose** | **Reference** |
|---|---|---|
| *E. coli* BL21 DE3 NudE.1 WT | Overexpression of His-tagged NudE.1 WT via pET28-NudE.1 WT | This study |
| *E. coli* BL21 DE3 NudE.1 M1 | Overexpression of His-tagged NudE.1 M1 via pET28-NudE.1 M1 | This study |
| *E. coli* BL21 DE3 NudC WT | Overexpression of His-tagged NudC WT via pET28-NudC WT | ¹ |
| *E. coli* BL21 DE3 NudC M1 | Overexpression of His-tagged NudC M1 via pET28-NudC M1 | ¹ |
| *E. coli* BL21 DE3 rL2 | Overexpression of His-tagged rL2 via pET28-rL2 | ⁷ |
| *E. coli* BL21 DE3 ModB | Overexpression of His-tagged ModB via pET28-ModB | ⁷ |
| Bacteriophage T4 WT | WT T4 phage | DSMZ |

**Table 2: DNA sequences of primers used.**

| **Name** | **Sequence** |
|---|---|
| NudE.1 fwd Ncol | ATCGACCCATGGGACAGGAAATTAAAATGAAAACATTATCAGC |
| NudE.1 revXhol | GTGCTCGAGGCCCTGAAAATAAAGATTCTCACCAAAGAGGTTGTTCATT ATTCGGTAAAG |
| NudE.1 E64,65Q fwd | CGAAGAGAATGTTTACAACAGACTGGTTTTAGC |
| NudE.1 E64,65Q rev | TGCTGCATCTAATGCGCTTAAATC |
| Fwd ultramer IVT Qbeta | TAATACGACTCACTATTATCTTGATACTACCTTTAGTTCGTTTAAACACGT TCTTGATAGTATCTTTTTATTAACC |
| Rev ultramer IVT Qbeta | CATGATCAAATTGACCCAAAGTTTCAACGCTTTACGCGTTGGGTTAATAA AAAGATACTATCAAGAACGTG |
| Fwd primer IVT Qbeta | TAATACGACTCACTATTATCTTGATACTACCTTTAG |
| Rev primer IVT Qbeta | CATGATCAAATTGACCCAAAGTTTCAACGCTTTACGCG |
| Fwd ultramer IVT RNAI | TAATACGACTCACTATTACAGTATTTGGTATCTGCGCTCTGCTGAAGCCA GTTACCTTCGGAAAAAGAGTTGGTAGCTCTTG |
| Rev ultramer IVT RNAI | AACAAAAAAACCACCGCTACCAGCGGTGGTTTGTTTGCCGGATCAAGA GCTACCAACTCTTTTTCCGAAGGTAACTGGCTTC |
| Fwd primer IVT RNAI | TAATACGACTCACTATTACAG |
| Rev primer IVT RNAI | AACAAAAAAACCACCGCTACC |

**Table 3: RNA sequences used in this study.**

| **Name** | **Sequence** |
|---|---|
| Qbeta-RNA | AUCUUGAUACUACCUUUAGUUCGUUUAAACACGUUCUUGAUAGUAUC UUUUUAUUAACCCAACGCGUAAAGCGUUGAAACUUUGGGUCAAUUU GAUCAUG |
| RNAI | ACAGUAUUUGGUAUCUGCGCUCUGCUGAAGCCAGUUACCUUCGGAA AAAGAGUUGGUAGCUCUUGAUCCGGCAAACAAACCACCGCUGGUAG CGGUGGUUUUUUUGUU |
| 10mer-Cy5 | pACAGUAUUUG-Cy5 |
| DNA-40mer-Cy5 | pACAGUAUUUGGUAUCUGCGCUCUGCUGAAGCCAGUUACCU-Cy5 |

**Table 4: DNA sequences of proteins as expressed from plasmids used in this study.**

| **Plasmid Name** | **Sequence** | **Reference** |
|---|---|---|
| pET28-NudE.1 WT | ATGGGACAGGAAATTAAAATGAAAACATTATC AGCTGGTATTATCTTTATGACAGAAGATAAAG ATTTATTTATGGGTCGGGTTACTGGTTCTCGT AAGACTGGAATGATGGCACATCGTTGGGATA TTCCAAAGGGCCGTGTAGAAAATTCTGATTT AAGCGCATTAGATGCAGCACGAAGAGAATGT TTAGAAGAGACTGGTTTTAGCAATTATAATCC AGACCTTCTAGAAGACCTAGGTGTATTTAAAT ATTCTAGTAATAAAGACCTACAGTTATTTTATT ACACGATTCCAGTAGAGCATGAGATGTTCAG AAATTGCCGTTGCGAGTCTTATTTTGAAAATA AAGATGGCGTTATGATTCCAGAGATGGACGC TTTTGCTCTTATTCCTCGTACTCAGTGGCAAT ATGTGATGGGTCCTTCACTTTACCGAATAATG AACAACCTCTTTGGTGAGAATCTTTATTTTCA GGGCCTCGAGCACCACCACCACCACCACTG A | This study |
| pET28-NudE.1 M1 | ATGGGACAGGAAATTAAAATGAAAACATTATC AGCTGGTATTATCTTTATGACAGAAGATAAAG ATTTATTTATGGGTCGGGTTACTGGTTCTCGT AAGACTGGAATGATGGCACATCGTTGGGATA TTCCAAAGGGCCGTGTAGAAAATTCTGATTT AAGCGCATTAGATGCAGCACGAAGAGAATGT TTACAACAGACTGGTTTTAGCAATTATAATCC AGACCTTCTAGAAGACCTAGGTGTATTTAAAT | This study |
| | ATTCTAGTAATAAAGACCTACAGTTATTTTATT ACACGATTCCAGTAGAGCATGAGATGTTCAG AAATTGCCGTTGCGAGTCTTATTTTGAAAATA AAGATGGCGTTATGATTCCAGAGATGGACGC TTTTGCTCTTATTCCTCGTACTCAGTGGCAAT ATGTGATGGGTCCTTCACTTTACCGAATAATG AACAACCTCTTTGGTGAGAATCTTTATTTTCA GGGCCTCGAGCACCACCACCACCACCACTG A | |
| pET28-NudC WT | ATGGATCGTATAATTGAAAAATTAGATCACGG CTGGTGGGTCGTCAGCCATGAACAAAAATTA TGGTTGCCGAAGGGAGAATTGCCATATGGC GAAGCGGCAAATTTCGATCTTGTGGGTCAG CGCGCACTACAAATCGGCGAATGGCAGGGG GAACCTGTTTGGTTAGTACAACAGCAGCGG CGTCACGATATGGGGTCGGTACGTCAGGTC ATTGATCTCGATGTTGGGCTGTTTCAACTGG CCGGACGAGGCGTACAACTGGCGGAGTTTT ACCGATCGCATAAATACTGTGGTTACTGCGG GCATGAAATGTATCCGAGCAAAACCGAATGG GCGATGCTGTGCAGCCATTGCCGTGAGCGT TACTACCCGCAAATCGCCCCCTGCATTATTG TTGCCATCCGTCGCGATGATTCGATCCTCCT CGCCCAGCATACCCGCCATCGTAACGGTGT CCATACAGTACTTGCCGGATTCGTCGAAGTG GGCGAAACCCTCGAGCAGGCAGTCGCGCG GGAAGTGATGGAAGAGAGCGGAATTAAAGT TAAAAACTTGCGTTACGTGACTTCTCAGCCG TGGCCGTTTCCTCAGTCTTTAATGACCGCGT TTATGGCGGAATATGACAGCGGCGACATCGT GATCGACCCGAAAGAATTGCTCGAGGCGAA CTGGTATCGCTATGACGATTTGCCGTTACTC CCGCCGCCCGGCACCGTAGCGCGCCGTCT GATAGAAGATACGGTGGCGATGTGTCGGGC AGAGTATGAGCTGGTGCCGCGCGGCAGCG CGGCCGCACTCGAGCACCACCACCACCAC CAC | ¹ |
| pET28-NudC M1 | ATGGATCGTATAATTGAAAAATTAGATCACGG CTGGTGGGTCGTCAGCCATGAACAAAAATTA TGGTTGCCGAAGGGAGAATTGCCATATGGC | ¹ |
| | GAAGCGGCAAATTTCGATCTTGTGGGTCAG CGCGCACTACAAATCGGCGAATGGCAGGGG GAACCTGTTTGGTTAGTACAACAGCAGCGG CGTCACGATATGGGGTCGGTACGTCAGGTC ATTGATCTCGATGTTGGGCTGTTTCAACTGG CCGGACGAGGCGTACAACTGGCGGAGTTTT ACCGATCGCATAAATACTGTGGTTACTGCGG GCATGAAATGTATCCGAGCAAAACCGAATGG GCGATGCTGTGCAGCCATTGCCGTGAGCGT TACTACCCGCAAATCGCCCCCTGCATTATTG TTGCCATCCGTCGCGATGATTCGATCCTCCT CGCCCAGCATACCCGCCATCGTAACGGTGT CCATACAGTACTTGCCGGATTCGTCGAAGTG GGCGAAACCCTCGAGCAGGCAGTCGCGCG GGAAGTGATGGAACAGAGCGGAATTAAAGT TAAAAACTTGCGTTACGTGACTTCTCAGCCG TGGCCGTTTCCTCAGTCTTTAATGACCGCGT TTATGGCGGAATATGACAGCGGCGACATCGT GATCGACCCGAAAGAATTGCTCGAGGCGAA CTGGTATCGCTATGACGATTTGCCGTTACTC CCGCCGCCCGGCACCGTAGCGCGCCGTCT GATAGAAGATACGGTGGCGATGTGTCGGGC AGAGTATGAGCTGGTGCCGCGCGGCAGCG CGGCCGCACTCGAGCACCACCACCACCAC CAC | |

**Table 5: Amino acid sequences of proteins as expressed from plasmids used in this study.**

| **Plasmid Name** | **Sequence** | **Reference** |
|---|---|---|
| pET28-NudE.1 WT | MGQEIKMKTLSAGIIFMTEDKDLFMGRVTGSR KTGMMAHRWDIPKGRVENSDLSALDAARREC LEETGFSNYNPDLLEDLGVFKYSSNKDLQLFY YTIPVEHEMFRNCRCESYFENKDGVMIPEMD AFALIPRTQWQYVMGPSLYRIMNNLFGENLYF QGLEHHHHHH | This study |
| pET28-NudE.1 M1 | MGQEIKMKTLSAGIIFMTEDKDLFMGRVTGSR KTGMMAHRWDIPKGRVENSDLSALDAARREC LQQTGFSNYNPDLLEDLGVFKYSSNKDLQLFY YTIPVEHEMFRNCRCESYFENKDGVMIPEMD AFALIPRTQWQYVMGPSLYRIMNNLFGENLYF QGLEHHHHHH | This study |
| pET28-NudC WT | MDRIIEKLDHGWWVSHEQKLWLPKGELPYG EAANFDLVGQRALQIGEWQGEPVWLVQQQR RHDMGSVRQVIDLDVGLFQLAGRGVQLAEFY RSHKYCGYCGHEMYPSKTEWAMLCSHCRER YYPQIAPCIIVAIRRDDSILLAQHTRHRNGVHTV LAGFVEVGETLEQAVAREVMEESGIKVKNLRY VTSQPWPFPQSLMTAFMAEYDSGDIVIDPKEL LEANWYRYDDLPLLPPPGTVARRLIEDTVAMC RAEYELVPRGSAAALEHHHHHH | ¹ |
| pET28-NudC M1 | MDRIIEKLDHGWWVSHEQKLWLPKGELPYG EAANFDLVGQRALQIGEWQGEPVWLVQQQR RHDMGSVRQVIDLDVGLFQLAGRGVQLAEFY RSHKYCGYCGHEMYPSKTEWAMLCSHCRER YYPQIAPCIIVAIRRDDSILLAQHTRHRNGVHTV LAGFVEVGETLEQAVAREVMEQSGIKVKNLRY VTSQPWPFPQSLMTAFMAEYDSGDIVIDPKEL LEANWYRYDDLPLLPPPGTVARRLIEDTVAMC RAEYELVPRGSAAALEHHHHHH | ¹ |

### References in the Introduction

1 Schauerte, M., Pozhydaieva, N. & Höfer, K. Shaping the Bacterial Epitranscriptome-5'-Terminal and Internal RNA Modifications. Adv Biol (Weinh) 5, e2100834 (2021). https://doi.org:10.1002/adbi.202100834
2 Shi, H., Chai, P., Jia, R. & Fan, X. Novel insight into the regulatory roles of diverse RNA modifications: Re-defining the bridge between transcription and translation. Mol Cancer 19, 78 (2020). https://doi.org:10.1186/s12943-020-01194-6
3 Sherwood, A. V. et al. Hepatitis C virus RNA is 5'-capped with flavin adenine dinucleotide. Nature 619, 811-818 (2023). https://doi.org:10.1038/s41586-023-06301-3
4 Cahova, H., Winz, M. L., Höfer, K., Nübel, G. & Jäschke, A. NAD captureSeq indicates NAD as a bacterial cap for a subset of regulatory RNAs. Nature 519, 374-377 (2015). https://doi.org:10.1038/nature14020
5 Chen, Y. G., Kowtoniuk, W. E., Agarwal, I., Shen, Y. & Liu, D. R. LC/MS analysis of cellular RNA reveals NAD-linked RNA. Nat Chem Biol 5, 879-881 (2009). https://doi.org:10.1038/nchembio.235
6 Wang, J. et al. Quantifying the RNA cap epitranscriptome reveals novel caps in cellular and viral RNA. Nucleic Acids Res 47, e130 (2019). https://doi.org:10.1093/nar/gkz751
7 Wolfram-Schauerte, M. & Höfer, K. NAD-capped RNAs - a redox cofactor meets RNA. Trends Biochem Sci 48, 142-155 (2023). https://doi.org:10.1016/j.tibs.2022.08.004
8 Hu, H. et al. SPAAC-NAD-seq, a sensitive and accurate method to profile NAD(+)-capped transcripts. Proc Natl Acad Sci USA 118 (2021). https://doi.org:10.1073/pnas.2025595118
9 Zhang, H. et al. Use of NAD tagSeq II to identify growth phase-dependent alterations in E. coli RNA NAD(+) capping. Proc Natl Acad Sci U S A 118 (2021). https://doi.org:10.1073/pnas.2026183118
10 Morales-Filloy, H. G. et al. The 5' NAD Cap of RNAIII Modulates Toxin Production in Staphylococcus aureus Isolates. J Bacteriol 202 (2020). https://doi.org:10.1128/JB.00591-19
11 Frindert, J. et al. Identification, Biosynthesis, and Decapping of NAD-Capped RNAs in B. subtilis. Cell Rep 24, 1890-1901 e1898 (2018). https://doi.org:10.1016/j.celrep.2018.07.047
12 Gomes-Filho, J. V. et al. Identification of NAD-RNAs and ADPR-RNA decapping in the archaeal model organisms <em>Sulfolobus acidocaldarius</em> and <em>Haloferax volcanii</em>. bioRxiv, 2022.2011.2002.514978 (2022). https://doi.org:10.1101/2022.11.02.514978
13 Ruiz-Larrabeiti, O. et al. NAD+ capping of RNA in Archaea and Mycobacteria. bioRxiv, 2021.2012.2014.472595 (2021). https://doi.org:10.1101/2021.12.14.472595
14 Jiao, X. et al. 5' End Nicotinamide Adenine Dinucleotide Cap in Human Cells Promotes RNA Decay through DXO-Mediated deNADding. Cell 168, 1015-1027 e1010 (2017). https://doi.org:10.1016/j.cell.2017.02.019
15 Bird, J. G. et al. The mechanism of RNA 5' capping with NAD+, NADH and desphospho-CoA. Nature 535, 444-447 (2016). https://doi.org:10.1038/nature18622
16 Chen, T. H., Potapov, V., Dai, N., Ong, J. L. & Roy, B. N(1)-methyl-pseudouridine is incorporated with higher fidelity than pseudouridine in synthetic RNAs. Sci Rep 12, 13017 (2022). https://doi.org:10.1038/s41598-022-17249-1
17 Miller, E. S. et al. Bacteriophage T4 genome. Microbiol Mol Biol Rev 67, 86-156, table of contents (2003). https://doi.org:10.1128/MMBR.67.1.86-156.2003
18 Luke, K. et al. Microarray analysis of gene expression during bacteriophage T4 infection. Virology 299, 182-191 (2002). https://doi.org:10.1006/viro.2002.1409
19 Wolfram-Schauerte, M., Pozhydaieva, N., Viering, M., Glatter, T. & Höfer, K. Integrated Omics Reveal Time-Resolved Insights into T4 Phage Infection of E. coli on Proteome and Transcriptome Levels. Viruses-Basel 14, 2502 (2022). https://doi.org:ARTN 250210.3390/v14112502
20 Wolfram-Schauerte, M. et al. A viral ADP-ribosyltransferase attaches RNA chains to host proteins. Nature 620, 1054-1062 (2023). https://doi.org:10.1038/s41586-023-06429-2

### References in the Examples

1 Höfer, K. et al. Structure and function of the bacterial decapping enzyme NudC. Nat Chem Biol 12, 730-734 (2016). https://doi.org:10.1038/nchembio.2132
2 Wolfram-Schauerte, M. & Höfer, K. NAD-capped RNAs - a redox cofactor meets RNA. Trends Biochem Sci 48, 142-155 (2023). https://doi.org:10.1016/j.tibs.2022.08.004
3 Xu, W., Gauss, P., Shen, J., Dunn, C. A. & Bessman, M. J. The gene e.1 (nudE.1) of T4 bacteriophage designates a new member of the Nudix hydrolase superfamily active on flavin adenine dinucleotide, adenosine 5'-triphospho-5'-adenosine, and ADP-ribose. J Biol Chem 277, 23181-23185 (2002). https://doi.org:10.1074/jbc.M203325200
4 Mirdita, M. et al. ColabFold: making protein folding accessible to all. Nat Methods 19, 679-682 (2022). https://doi.org:10.1038/s41592-022-01488-1
5 Höfer, K., Abele, F., Schlotthauer, J. & Jäschke, A. Synthesis of 5'-NAD-Capped RNA. Bioconjugate Chem 27, 874-877 (2016). https://doi.org:10.1021/acs.bioconjchem.6b00072
6 Ramanathan, A., Robb, G. B. & Chan, S. H. mRNA capping: biological functions and applications. Nucleic Acids Res 44, 7511-7526 (2016). https://doi.org:10.1093/nar/gkw551
7 Wolfram-Schauerte, M. et al. A viral ADP-ribosyltransferase attaches RNA chains to host proteins. Nature 620, 1054-1062 (2023). https://doi.org:10.1038/s41586-023-06429-2
8 Nübel, G., Sorgenfrei, F. A. & Jäschke, A. Boronate affinity electrophoresis for the purification and analysis of cofactor-modified RNAs. Methods 117, 14-20 (2017). https://doi.org:10.1016/j.ymeth.2016.09.008

## Claims

1. A method for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein the method comprises contacting the 5'-dinucleotide-capped nucleic acid molecule with
(I)
an enzyme being capable of cleaving the diphosphate, triphosphate or tetraphosphate linkage, wherein the enzyme
(a) comprises or consists of the amino acid sequence of SEQ ID NO: 21,
(b) comprises or consists of an amino acid sequence being at least 80%, preferably at least 90% and most preferably at least 95% identical to SEQ ID NO: 21,
(c) comprises or consists of the amino acid sequence being encoded by the nucleotide sequence of SEQ ID NO: 17, or
(d) comprises or consists of an amino acid sequence being encoded by a nucleotide sequence being at least 80%, preferably at least 90% and most preferably at least 95% identical to SEQ ID NO: 17;
(II)
a nucleic acid molecule, preferably a vector, encoding in expressible form the enzyme of (I); and/or
(III)
a host cell comprising the nucleic acid molecule, preferably the vector of (II).

2. Use of an enzyme, nucleic acid molecule and/or a host cell for decapping a 5'-dinucleotide-capped nucleic acid molecule, wherein the dinucleotide is linked to the nucleic acid molecule via a phosphodiester bond and the dinucleotide comprises a diphosphate, triphosphate or tetraphosphate linkage between the two nucleotides of the dinucleotide, and wherein
(I) the enzyme being capable of cleaving the diphosphate, triphosphate or tetraphosphate linkage,
(a) comprises or consists of the amino acid sequence of SEQ ID NO: 21,
(b) comprises or consists of an amino acid sequence being at least 80%, preferably at least 90% and most preferably at least 95% identical to SEQ ID NO: 21,
(c) comprises or consists of the amino acid sequence being encoded by the nucleotide sequence of SEQ ID NO: 17, or
(d) comprises or consists of an amino acid sequence being encoded by a nucleotide sequence being at least 80%, preferably at least 90% and most preferably at least 95% identical to SEQ ID NO: 17;
(II) the nucleic acid molecule, preferably vector, encodes in expressible form the enzyme of (I); and/or
(III) the host cell comprises the nucleic acid molecule, preferably the vector of (II).

3. A kit for decapping a 5'-dinucleotide-capped nucleic acid molecule, wherein the dinucleotide is linked to the nucleic acid molecule via a diphosphate and the dinucleotide comprises a diphosphate, triphosphate or tetraphosphate linkage between the two nucleotides of the dinucleotide, and wherein the kit comprises
(I)
an enzyme being capable of cleaving the diphosphate, triphosphate or tetraphosphate linkage, wherein the enzyme
(a) comprises or consists of the amino acid sequence of SEQ ID NO: 21,
(b) comprises or consists of an amino acid sequence being at least 80%, preferably at least 90% and most preferably at least 95% identical to SEQ ID NO: 21,
(c) comprises or consists of the amino acid sequence being encoded by the nucleotide sequence of SEQ ID NO: 17, or
(d) comprises or consists of an amino acid sequence being encoded by a nucleotide sequence being at least 80%, preferably at least 90% and most preferably at least 95% identical to SEQ ID NO: 17;
(II)
a nucleic acid molecule, preferably a vector, encoding in expressible form the enzyme of (I); and/or
(III)
a host cell comprising the nucleic acid molecule, preferably the vector of (II).

4. The method of claim 1, the use of claim 2, or the kit of claim 3, wherein the dinucleotide is 7-methyl-guanosine-5'-triphosphate-5'-guanosine, diadenosine tetraphosphate, NXD or FXD, wherein X is a nucleobase, preferably a purine or pyrimidine nucleobase, more preferably adenine, guanine, cytosine, thymine, or uracil and most preferably adenine.

5. The method, use, or kit of any one of claims 1 to 4, wherein the dinucleotide-capped nucleic acid molecule is a dinucleotide-capped RNA molecule.

6. The method, use, or kit of any one of claims 1 to 5, wherein the enzyme being capable of cleaving the diphosphate, triphosphate or tetraphosphate linkage comprises the sequence GXXXXXEXXXXXUAXREXXEEXGU (SEQ ID NO: 25), wherein X can be any amino acid and U is a bulky, hydrophobic amino acid, preferably Phe, Ile, Leu, or Val.

7. The method, use, or kit of claim 6, wherein the enzyme being capable of cleaving the diphosphate, triphosphate or tetraphosphate linkage comprises the sequence GRVENSDLSALDAARRECLEETGF (SEQ ID NO: 26) or a sequence being at least 80%, preferably at least 90% and most preferably at least 95% identical thereto, provided that the underlined amino acids are retained.

8. The method, use, or kit of any one of claims 1 to 7, wherein in the method or use the decapping is carried out in or the kit further comprises 0.5 mM to 2mM DTT, preferably about 1 mM DTT and a degradation buffer, wherein the degradation buffer comprises
10 to 50 mM, preferably about 25 mM Tris-HCl pH 7.5,
25 to 100 mM NaCl, preferably about 50 mM NaCl,
25 to 100 mM KCI, preferably about 50 mM KCI, and
2.5 to 20 MgCl₂, preferably about 10 mM MgCl₂.

9. The method or use of any one of claims 1 to 8, further comprising the degradation of the uncapped nucleic acid molecule by a RNase and/or DNase.

10. The kit of any one of claims 1 to 8, further comprising RNase and/or DNase for degrading the uncapped nucleic acid molecule.

11. The method or use of any one of claims 1 to 9, further comprising prior to the decapping attaching a dinucleotide to the 5'-end of a nucleic acid molecule.

12. The method or use of any one of claims 1 to 9, further comprising prior to the decapping isolating a 5'-dinucleotide-capped nucleic acid molecule from a total RNA sample, preferably by NAD captureSeq, NADcapPro or CapzymeSeq.

13. The method, use, or kit of any preceding claim, wherein the 5'-dinucleotide-capped nucleic acid molecule is covalently attached to a protein or peptide.

14. The method or use of claim 13, wherein prior to the decapping the 5'-dinucleotide-capped nucleic acid molecule is attached to the protein or peptide by an ADP-ribosyltransferase (ART), wherein the ART preferably comprises or consists of SEQ ID NO: 27 or SEQ ID NO: 28 or a sequence being at least 80% identical thereto.

15. The kit of claim 13, wherein the kit further comprises an ART for attaching the 5'-dinucleotide-capped nucleic acid molecule to the protein or peptide, wherein the ART preferably comprises or consists of SEQ ID NO: 27 or SEQ ID NO: 28 or a sequence being at least 80% identical thereto.
